# EUROPEAN PATENT APPLICATION

(11) **EP 1 852 107 A1**
(43) Date of publication of application: **07.11.2007**
(21) Application number: 06290638.3
(22) Date of filing: 19.04.2006
(51) Int. Cl.: A61K 9/14, A61K 49/06, A61K 33/26, A61P 35/00

(54) **Magnetic nanoparticles compositions and uses thereof**

(71) Applicant: Nanobiotix, 75002 Paris (FR)
(72) Inventor: Levy, Laurent, 75015 Paris (FR); Germain, Matthieu, 94800 Villejuif (FR); Devaux, Corinne, 78330 Fontenay Le Fleury (FR)
(74) Representative: Gallois, Valérie

(57) **Abstract**

The present invention relates to the use of a biocompatible nanoparticle or nanoparticle aggregate, in combination with an external non-oscillating magnetic field, wherein said nanoparticle comprises a) a core comprising magnetic material; b) a biocompatible shell surrounding the core, and, optionally, c) a labelling agent, wherein the outer diameter of the shell is less than about 100 nm, to prepare a composition, wherein the composition is deprived of any cell targeting means. The present invention further relates to the compositions thus obtained and to their uses in the field of human health, for the treatment of cancer, or in diagnostic (imaging for example), for the monitoring of tumor evolution.

## Description

The present invention relates generally to the area of activable particles, for cancer therapy and tumor evolution monitoring. More particularly, the present invention provides pharmaceutical compositions comprising biocompatible magnetic nanoparticles, methods for preparing said compositions and using them in the treatment of cancer or in imaging, for the monitoring of tumor evolution, using a non-oscillating magnetic field.

### BACKGROUND OF THE INVENTION

In the past 30 years, major advances have been made in the diagnosis and treatment of human malignancies. In parallel, biotechnologies and nanotechnologies have provided avenues for the development of novel approaches to treatment of human diseases. Currently, chemotherapy is a widely used method of treatment for many cancers, but it has some distinct limitations and disadvantages. The main disadvantage of chemotherapy is related to drug toxicity, which severely restricts the drug dosages that can be used to effect eradication of the cancer cells.

Since 1950, magnetic probes and particles have been investigated as a potential treatment for cancer. Studies demonstrate that the hyperthermia (Grittner et al., 1997, Hybridoma, 16:109; Higler et al., 1997, Invest. Radiol., 32:705) generated by magnetic particles coupled to a hyper frequency (HF) alternating current (AC) magnetic field (requiring a tremendous power) could be used as an alternate or an adjuvant to therapy for cancer treatment. The hyperthermic activity (heat produced by the relaxation magnetic energy of the magnetic material) was shown to effectively destroy tumor tissue surrounding the probes or particles. The development of ultrasmall magnetic particles (ferrofluids) with high crystallinity provided the next step in magnetically induced hyperthermic therapy. This treatment resulted in reduction of tumor size when the particles were directly injected into the tissue and exposed to an alternating magnetic field. When using this kind of treatment, side effects due to potential interactions between radiations and tissues cannot however be excluded.

Furthermore, the cost and poor availability of HF devices (not currently existing in health care system) are a limitation regarding their uses in therapy.

Specific targeting of chemotherapeutic objects to the cancer cells has been another area of research (Schally et al., 1999, J. Endocrinol., 141:1; Nagy et al., 1996, Proc. Natl. Acad. Sci., USA, 93:7269; Emons et al., 1993, J. Clin. Endocrinol. Metab., 77:1458). The approach of targeted specific therapy was combined with nanotechnology to demonstrate the concept of nanoclinics as a novel anti-cancer approach. US 6,514,481 describes the use of non-oscillating magnetic field to effect the magnetocytolysis of selective LH-RH receptor-positive cancer cells targeted by magnetic iron oxyde particles containing LH-RH as a targeting agent. Bergey et al. (Biomedical Microdevices 4:4, 293-299, 2002) and Levy et al. (Chem. Mater. 2002, 14, 3715-3721) not only supported the selective targeting action of such nanoclinics but specified that control cells lacking the receptor for LH-RH did not show the binding or accumulation or nanoclinics and were therefore not sensitive to an exposure to the magnetic field.

This approach although efficient to destroy a specific tumor cell type, requires money- and time-consuming preparation of nanoclinics designed to target said specific cell type. One type of particles is indeed only usable for one type of cancer cell. Therefore multiple nanoparticles and developments are needed to address different cancers.

### SUMMARY OF THE INVENTION

The present inventors have now discovered that nanoparticles having a core of a therapeutic or diagnostic magnetic, preferably ferromagnetic, material, optionally surrounded by a shell composed of a biocompatible material, may be used in a pharmaceutical or diagnostic composition even when they are deprived of any cell targeting agent. Those nanoparticules are herein called "untargeted" nanoparticles.

The present invention relates generally to the use of biocompatible magnetic, preferably ferromagnetic, nanoparticles, to prepare compositions for various biological and therapeutic applications, and to the methods related thereto.

Inventors herein provide novel compositions comprising nanosized (less than about 100 nm) particles termed as "nanotherapeutics", "nanobiodrugs", "nanoclinics", "nanoparticles" or "nanobubbles" for therapeutic use or tumor evolution monitoring.

Inventors more specifically provide new compositions comprising magnetic, preferably ferromagnetic, nanoparticles, which may be used, in therapy, to prevent or treat a cancer, or in diagnostic (for example in *in vivo* imaging), to monitor the tumor evolution (growth or regression).

As indicated above, the nanoparticles have a core of a therapeutic or diagnostic magnetic material optionally surrounded by a shell composed of a biocompatible material. The nanoparticles optionally contain a labelling agent.

A particular feature of these nanoparticles is that they are deprived of any cell targeting agent.

In therapy, nanoparticles are preferably used, in the present invention, in combination with an external magnetic field, to prepare a pharmaceutical composition to prevent or treat a cancer.

The pharmaceutical compositions provided by the present invention are thus deprived of any cell targeting agent and comprise a nanoparticle comprising a) a core comprising magnetic material, preferably ferromagnetic material, and optionally b) a biocompatible layer surrounding the core, wherein the outer diameter of the shell is less than about 100 nm, preferably less than about 50 nm, in association with a biocompatible carrier.

A method for the selective destruction of targeted cells, such as cancer cells, is in particular herein described. Upon exposure of the cells to nanoclinics having a magnetic core, tumor will attach and/or internalize the nanoclinics. Subsequent application of a non-oscillating or stable magnetic field can specifically destroy the targeted cells.

In imaging, nanoparticles are preferably used, in the present invention, in combination with an external magnetic field as described above, to prepare a monitoring composition intended for the detection or the visualization of a cancer cell, tissue or organ, in the absence of any cell targeting agent.

The present invention thus further provides diagnostic compositions useful to monitor the tumor growth or regression.
These compositions, also deprived of any cell targeting agent, comprise a nanoparticle comprising a) a core comprising magnetic material, preferably ferromagnetic material; optionally b) a biocompatible shell surrounding the core, and c) a labelling agent, wherein the outer diameter of the shell is less than about 100 nm, preferably less than about 50 nm, in association with a biocompatible carrier.

### DETAILED DESCRIPTION OF THE INVENTION

The terms "nanotherapeutic", "nanobiodrug", "nanobubble", "nanoparticle", "nanoclinic" or plurals thereof, are used interchangeably and, for the purposes of the specification, mean, a biocompatible particle or aggregate of particles of less than about 100 nm diameter, deprived of any cell targeting agent, having a central core of therapeutic or diagnostic material, optionally a shell surrounding the central core; and optionally, a labelling agent.

The terms "magnetic nanoparticle", "nanoMAG", "magnetic agent", or "magnetic material", are used interchangeably and, as used herein, refers to a magnetic, in particular ferromagnetic, energy susceptive untargeted particle or aggregate of particles that optionally comprises a biocompatible coating and that, when exposed to an external magnetic field source, takes a preferential orientation implying a physical rotation leading to the therapeutic effect.

The term "magnetic nanoparticle composition", as used herein, refers to a composition, deprived of any cell targeting agent, that comprises a magnetic, preferably ferromagnetic, nanoparticle, and a suitable medium.

The term "treatment" indicates herein any improvement of the pathological signs, like, in particular, a reduction in the size or development of a tumour, the removal or the destruction of pathological cells or tissues, a deceleration or stabilization of the progression of the cancer, a reduction of the formation of metastases, a regression or a complete remission, etc.

The particles according to the invention can be implemented to mark, deteriorate or destroy cells, tissues or organs in combination with an external magnetic field *in vitro, in vivo* or ex *vivo.* Particles and compositions comprising said particles according to the invention, may be used on any type of tissue, either superficial or deep, in any mammalian organism, preferably in a human.

An object of the present invention relates to the use of a biocompatible nanoparticle or nanoparticle aggregate, in combination with an external magnetic field, wherein said nanoparticle comprises:
a) a core comprising magnetic, preferably ferromagnetic, material; optionally
b) a biocompatible shell surrounding the core, wherein the outer diameter of the shell is less than about 100 nm, preferably less than about 50 nm; and optionally
c) a labelling agent, that can be linked to the magnetic material or to the biocompatible layer,
   for the manufacture of a pharmaceutical composition, to prevent or treat a cancer,
wherein the composition is deprived of any cell targeting means.

In another embodiment, the present invention relates to the use of a biocompatible nanoparticle or nanoparticle aggregate, in combination with an external magnetic field, wherein said nanoparticle comprises:
a) a core comprising magnetic, preferably ferromagnetic, material; optionally
b) a biocompatible shell surrounding the core, wherein the outer diameter of the shell is less than about 100 nm, preferably less than about 50 nm; and
c) a labelling agent, that can be linked to the magnetic material or to the biocompatible layer,
   for the manufacture of a monitoring composition intended for the detection or the visualization of a cancer cell, tissue or organ, wherein the composition is deprived of any cell targeting means.

The biocompatible magnetic particles useful for the present invention need to be small enough in size to be able to diffuse into the tissue to enter the cells (by endocytotic processes) without being captured by macrophages (by phagocytosis) and large enough to respond to the applied magnetic field at 37.degree. C. Thus, particles less than 200 nm, preferably less than about 100 nm in diameter, or in the range of about 10 nm to less than about 100 nm in diameter, even more preferably particles less than 50 nm in diameter, are suitable for the present invention. Preferred particles useful for the present invention have a diameter comprised between about 10 and about 50 nm.
Magnetic core should be large enough to present magnetic properties, in particular ferromagnetic properties, in order to provide a therapeutic or diagnostic effect.

The nanoparticles may have different shapes. They may for example be round, flattened, lengthened, spherical, oval, etc. The form can be determined or controlled by the manufacturing process, and be adapted by the man of the art according to the magnetic field to be applied and to the required applications.
The shape of the particles may have an influence on their properties (especially on magnetic properties). The shape can influence the "biocompatibility" and biodistribution of the particles. Thus, for reasons of pharmacokinetics and to avoid potential undesired interactions, nanoparticles or nanoparticle aggregates of essentially spherical or round shape are preferred. In addition, nanoparticles or nanoparticle aggregates of rather homogeneous form are preferred.

Nanoparticles usable in the present invention have to be biocompatible, i.e., when administered to an organism, typically a mammal, preferably a human, they should not induce any adverse effect. This biocompatible character can be assured for example by the nature of the compounds constitutive of the particle and/or by the nature of the coating when present.

The material forming the core may be one or more therapeutic or diagnostic magnetic material including at least one magnetic, preferably ferromagnetic, material. Such materials include iron, nickel, cobalt, gadolinium, samarium, neodymium, boron, aluminium, preferably in the form of an oxide, an hydroxide or a metal thereof, and any mixture thereof. In a preferred embodiment, the magnetic core material is a metallic material, preferably a ferromagnetic material, even more preferably a monodomain ferromagnetic material. In specific examples, the material forming the core is selected from the group consisting of ferrous oxide and ferric oxide.
Mixed material can be used to optimize interactions between a magnetic field and nanoparticles. Solid solution forms (well known by the man skilled in the art as random mixtures of several materials) such as CoFe₂O₄ for example can be used as a mixed material. Solid solution form in demixed phases, such as Fe₂O₃ / Co for example, can further be used.

As mentioned previously, nanoparticules or nanoparticule aggregates used in the present invention optionally comprises a shell. Such a coating advantageously makes it possible to preserve the integrity of the particles *in vivo* and to ensure or improve their biocompatibility and specific biodistribution.

The biocompatible shell can be made of any amorphous or crystalline material.
In a general way, coating can be non-biodegradable or biodegradable. Non biodegradable coatings may be selected from silica (SiO₂), gold (Au), agarose, alumina (Al₂O₃), a saturated carbon polymer and an inorganic, linear or branched polymer, modified or not (polystyrene for example). Biodegradable coatings may for example be selected from natural or artificial biological molecules which may be modified or not. It may be a polymer made of a biological molecule, modified or not, of natural form or not, or a biological polymer, such as saccharide, an oligosaccharide, a polysaccharide, polysulfated or not, for example the dextran. The materials or compounds thus mentioned can be used alone, in mixtures or assemblies, composites or not, covalent or not, possibly in combination with others compounds. In addition, one can also use any material mentioned above, hydro or liposoluble, in a natural or artificial way.
The bio-compatible material is preferably selected from, but not limited to, silica (SiO₂), alumina (Al₂O₃), Polyethylene Glycol (PEG) and dextran.

A labelling agent (also referred to herein as "labelling dye") may be used optionally to track the nanoparticles. This labelling agent may be attached to the magnetic material forming the core of the nanoparticle or to the biocompatible layer when present. The labelling agent may be inside the biocompatible layer or attached to the surface of the nanoparticle. The labelling agent may be selected from the group consisting of fluorescent markers derivatives, chemical dyes, ultrasound contrast agents, x-ray contrast agents and magnetic resonance imaging agents.

The labelling dye may be any fluorescent dye. An example is a dye that has an excitation wavelength in the infra red range. A description of two photon dyes that are suitable is found in U.S. Pat. No. 5,912,247.

In the present invention, nanoparticles can comprise other molecules, compounds, or surface or structural materials, intended to improve their stability, property, function, specificity, etc. It may be a molecule ensuring or improving the biocompatibility of the nanoparticle or a molecule allowing the nanoparticle to escape from the immune system (and in particular to avoid the interactions with the macrophages and reticulo-endothelial system). Said nanoparticles are however deprived of any specific cell targeting agent.

In one embodiment, the nanoparticles, used in the present invention, are structures comprising a core made of Fe₂O₃ or Fe₃O₄, optionally, an optical probe as the labelling agent, and preferably a silica shell as a biocompatible shell. A preferable size for the magnetic core is about 20 nm. It is considered that the silica shell stabilizes the magnetic core.

A typical method of preparing nanoparticles usable in the present invention, comprises the steps of:
a) forming a magnetic, preferably ferromagnetic, core with a material selected from the group described previously, for example with ferrous oxide or ferric oxide; and preferably
b) forming a biocompatible shell, for example a silica shell, around the core;
wherein the outer diameter of the silica shell is less than about 100 nm, preferably less than 50nm.

Particles with a magnetic core may be synthesized using a two-step process such as described in example 1.

Alternative methods of production of materials usable for the production of the nanoparticles usable in the present invention are described for example in US 6,514,481 B1, in Nelson et al, Chem. Mater. 2003, 15, 688-693 "Nanocrystalline Y203:Eu Phosphors Prepared by Alkalide Reduction*"* or in Liu et al., Journal of Magnetism and Magnetic Materials 270 (2004) 1-6 "Preparation and characterization of amino-silane modified superparamagnetic silica nanospheres*".*

From the teachings of the present invention, those skilled in the art will recognize that the untargeted magnetic nanoparticules may be modified without departing from the spirit of the invention.

Another object of the invention resides in any composition comprising the biocompatible untargeted magnetic nanoparticles or aggregate of nanoparticles such as defined previously and/or likely to be obtained by anyone of the processes described previously.

Although not compulsory, the nanoparticles of the compositions of the invention advantageously have a rather homogeneous form and size.
The compositions can be in solid or in liquid form (suspended nanoparticles), in the form of a paste, aerosol, etc.

In a particular embodiment, the present invention provides a pharmaceutical composition, deprived of any cell targeting agent, comprising a nanoparticle such as defined above, preferably in a therapeutically effective amount, comprising a) a core comprising magnetic material, preferably a core made of ferromagnetic material, for example of ferrous oxide or ferric oxide, and preferably b) a biocompatible shell surrounding the core, wherein the outer diameter of the shell is less than about 100 nm, preferably between about 10 nm and about 50 nm, in association with a biocompatible carrier.

The therapeutically effective amount may be between about 0,01 mg and about 100 mg/g of tumor, preferably between about 0,05 mg and about 30 mg/g of tumor, even more preferably between about 0,05 mg and about 10 mg/g of tumor. Size and weight of the tumour can be estimated and calculated using imaging MRI or Scanner.

In a further embodiment, the present invention provides a diagnostic or monitoring composition, deprived of any cell targeting agent, comprising a nanoparticle such as defined above, preferably in a diagnostically effective amount, comprising a) a core comprising magnetic material, preferably a core made of ferromagnetic material, for example of ferrous oxide or ferric oxide, preferably b) a biocompatible shell surrounding the core, and c) a labelling agent, wherein the outer diameter of the shell is less than about 100 nm, preferably between about 10 nm and about 50 nm, in association with a biocompatible carrier.

This diagnostic or monitoring composition may be combined with the pharmaceutical composition or assimilated to the pharmaceutical composition in particular when the monitoring and the treatment are realized simultaneously. In the latter situation, the same nanoparticles are generally used as a therapeutic and as a diagnostic tool.

The amount of nanoparticles used to prepare the diagnostic or monitoring composition will depend on the amount of nanoparticles used to provide a therapeutic effect.

The excipient or carrier can be any very usual support for this type of applications, such as for example saline, isotonic, sterile or buffered solutions, etc. They can further comprise stabilizing, sweetening and/or surface-active agents, etc. They can be formulated in the form of ampules, flasks, tablets, or capsules, by using techniques of galenic known per se.

The compositions, nanoparticles and aggregate of the invention can be used in many fields, particularly in human or animal medicine.

When exposed to a magnetic field, and depending on the duration of the exposure, magnetic nanoparticles allow the cell or tissue destruction (duration of several minutes, for example from 2 or 5 minutes to 120 minutes) or, simply, a monitoring or visualization thereof (imaging, diagnosis) (duration of several seconds or minutes, in particular from 1 second to 120 minutes, preferably from 1 minute to 60 minutes, for example from 10 seconds to 10 minutes). In particular, monitoring can be recorded during the treatment time.

Other imaging modalities such as scanner, mammography, PET, optical ultrasound can be used to visualize nanoparticles and provide imaging for diagnosis and/or tumor follow-up purpose.

Using magnetic fields, the particles of the invention are applicable for a scanning of any tissue in the body.

The nanoparticles or nanoparticule aggregates and compositions of the present invention can be advantageously used for lysis of cancer cells or cells suspected to be the same, when subjected to a magnetic field.

In a particular embodiment, the present invention thus relates to the use of a biocompatible nanoparticle or nanoparticle aggregate, in combination with an external magnetic field, wherein said nanoparticle comprises:
a) a core comprising magnetic, preferably ferromagnetic, material; and optionally
b) a biocompatible shell surrounding the core, wherein the outer diameter of the shell is less than about 100 nm, preferably around or less than about 50nm;
   for the manufacture of a pharmaceutical composition, to destruct a cell such as a cancer cell, in particular to prevent or treat a cancer, wherein the composition is deprived of any cell targeting means.

The invention further relates to a method of inducing or causing apoptosis, necrosis or lysis of a tumor cell *in vitro, ex vivo* or *in vivo,* comprising the steps of:
(a) contacting the pharmaceutical composition according to the invention and described previously with tumor cells for a time sufficient for said tumor cells to bind and/or internalize the nanoparticles or aggregate of nanoparticles contained in said pharmaceutical composition ; and
(b) exposing the cells to a non-oscillating or stable magnetic field,
wherein said exposure causes apoptosis, necrosis or lysis of the tumor cells which have bound and/or internalized the nanoparticles.

While not intending to be bound by any particular theory, the data, described in details in the experimental part of the present application, suggest that a probable mechanism for cytolysis could be a physical orientation of magnetic nanoparticles in the field, which induces a physical stress or local disruption leading to the cell destruction. The present invention demonstrates the magnetocytolytic ability (cell lysing) of these nanoclinics.

Evidence presented here suggests these nanoclinics interact with cancer cells and concentrate in tumors by escaping from the leaky vasculature surrounding solid tumors through a phenomenon known as the enhanced permeation and retention (EPR) effect. Tumor tissues are indeed known to have leaky vasculature and decreased lymphatics compared with normal histology. This phenomenon results in a passive accumulation of nanoscaled particles in tumors.

As explained previously, the cells to be destroyed may be any cancer cells from any mammal, in particular from a human. The cancer cells are preferably selected from the group consisting of colon cancer cells, breast cancer cells, ovarian cancer cells, pancreatic cancer cells, kidneys cancer cells, bladder cancer cells, oesophageal cancer cell, brain cancer cells, liver cancer cells, endometrial cancer cells, prostate cancer cells and pancreatic cancer cells.

Another object of the invention relates to a method for stabilizing or treating a cancer, comprising (a) the administration to a patient suffering of a cancer of a composition of the invention such as previously defined, under conditions allowing the nanoparticles or aggregate of nanoparticles included in said composition to contact or penetrate the cancer cells, and (b) the exposition of the patient to a magnetic field, as explained above, leading to a deterioration, a disturbance or a functional destruction of his cancer cells, thus treating his cancer.

The above described method is usable to treat any type of cancer, in particular the solid tumors, metastasized or not.
Human malignancies that may be stabilized or treated using magnetic nanoparticles according to the invention include, but are not limited to, colon, liver, lung, kidney, bladder, head-and-neck, brain, skin, intestine, breast, ovarian, endometrial, prostate, pancreatic cancer, etc.

Nanoparticles or compositions according to the invention as described above can be administered by various ways, preferably by injection. Injection may be local [intratumoral (IT) or peri-tumoral for example], to ensure a local concentration in the tumor and to maximize the therapeutic effect, or systemic [for example intra-veinous (IV)], to allow the passive accumulation of nanoparticles in the tumor through the EPR effect. Administration may also be realized in an oral way. Repeated injections or administrations can be considered, if necessary.

The magnetic field, which is preferably non-oscillating or stable, can be applied constantly after a first administration of the nanoparticles, in one or more time, by using any magnetic field source. Each activation by a magnetic field may be followed by one or several administrations of nanoparticles or compositions according to the invention.

The magnetic field source is preferably a uniform and unidirectional magnetic field source and may be selected from any permanent magnet, electromagnet and Magnetic Resonance Imaging (MRI) equipment.
A suitable non-oscillating or stable magnetic field is available in standard MRI equipment which typically has a magnetic filed in the range of 0.5 to 5 Tesla.

For effecting treatment or diagnosis, tumor cells are, as described previously, exposed to the nanoparticles or nanoparticle aggregates according to the invention. Nanoparticles or nanoparticle aggregates may be administered in one or more times, preferably in one time. Nanoparticles or nanoparticle aggregates, when administered in several times, for example 2, 3, 4 or 5 times, may be regularly administered during at least one week, preferably two weeks.
Following binding and/or internalization of the nanoparticles or nanoparticle aggregates by the tumor cells, the patient is exposed to a magnetic field, preferably to a non-oscillating or stable magnetic field.

For diagnosis purpose, the compositions of the invention are, as explained previously, also usable as contrast or diagnostic agents (monitoring compositions), to detect and/or visualize any type of cancer tissue.

Thus, an object of the invention relates to the use of nanoparticles or aggregate of nanoparticles such as previously described, in combination with a magnetic field, for the manufacture of a composition intended for the detection or the visualization of cells, tissues or organs, or for the monitoring of the evolution of the pathology.

The term "in combination" indicates that the required effect is obtained when the cells, tissues or organs of interest, having partly incorporated nanoparticles of the invention, are excited by the magnetic field. However, as explained above, in the context of therapy, it is not necessary that the particles and the magnetic field are managed simultaneously, nor according to the same protocol.

Such a composition may be used during the treatment step.

The magnetic field applied in therapy or diagnosis is in the range of 0.5 to 7 Tesla, preferably in the range of 0.5 to 5 Tesla. Typically an MRI device can be used to provide excitation of nanoparticles with a 1,5 Tesla magnetic field.
At an appropriate time (when biodistribution is suitable for good therapeutic activity and minimizing potential collateral damages), after a single or repeated administrations of nanoparticles or compositions of the invention, a magnetic field may be applied in one or more time on one or several weeks (preferably 1, 2, 3 or 4 weeks) and is preferably applied every 1 or 2 days. The number of activation is preferably inferior or equal to 5 [for example 1, 2, 3, 4 or 5 activation(s)] and typically lasts from 30 minutes to 90 minutes in therapy, and from 10 second to 60 minutes in diagnosis.

The protocol described previously can be repeated during time if needed (every month or every two , three, four, five or six months for example).

The invention will be appreciated better from the following examples, which are intended to be illustrative and not limiting.

### BRIEF DESCRIPTION OF THE DRAWINGS

FIG. 1 is a TEM picture of iron oxide nanoparticles coated with Silica.
FIG. 2 is a representation of the evolution of C57BL6 mice weight after injection of increasing doses of nanoparticles ranging from 0,006 to 0,06 mg of untargeted nanoMAG composition/mouse. Figure 1 demonstrates no effect on mice weight of the untargeted nanoMAG composition during the month following the administration thereof.
FIG. 3 is a representation of the evolution of C57BL6 mice weight after a single injection of 0,9 and 1,8 mg of untargeted NanoMAG composition/mouse. Figure 2 demonstrates no effect on mice weight of the untargeted nanoMAG composition.
FIG. 4 is a representation of the mice bearing C38 tumors, weight before and after 5 intravenous injections of untargeted nanoMAG composition (0.12mg/mouse). There is no difference between the weight of control mice and the weight of mice subjected to MRI.
FIG. 5 is a representation of the decrease of the C38 tumor growth in untargeted nanoMAG injected C57BI6 mice subjected to a magnetic field during 1 hour, 20 hours post-injection, compared to untargeted nanoMAG injected and not activated animals.
FIG. 6 is a representation of the evolution of the C57BI6 mice weight after injection of NaCl or injection and activation after 5, 20 or 48 hours of untargeted nanoMAG which reveals no difference between control and treated animals.
FIG. 7 is a representation of the tumor volumes of mice treated with activated untargeted NanoMAG relative to untreated mice. The growth of the tumors is decreased in mice treated with untargeted nanoMAG activated 48 hours after injection.
FIG. 8 is an MRI pictures of mice, bearing C38 tumors (a) after single IT injection of NaCl 0,9% solution and (b) 48 hours after single IT injection of nanoMag.

### EXAMPLE 1

Particles with a magnetic core are synthesized using a two-step process. A precipitation step is realized by simultaneous injection of aqueous solutions of ferrous chloride (60 mmoles) and sodium hydroxide (120 mmoles) in a reactor under nitrogen atmosphere, and mechanical agitation. Then, the obtained iron hydroxide solution is directly oxidized by injection of hydrogen peroxide in the reactor. During oxidation step, reaction bulk is kept at pH 8 using an auto-burette filled with a sodium hydroxide solution. During all this process, pH, temperature, and added volume of sodium hydroxide are recorded by a computer linked to the pH-meter. Solution is incubated for 2 hours after the end of hydrogen peroxide injection. Then the 5 g particles obtained (30 nm diameter) are washed by centrifugation in distilled water and stabilized by addition of 1.2 mM of tetramethylammonium hydroxide.

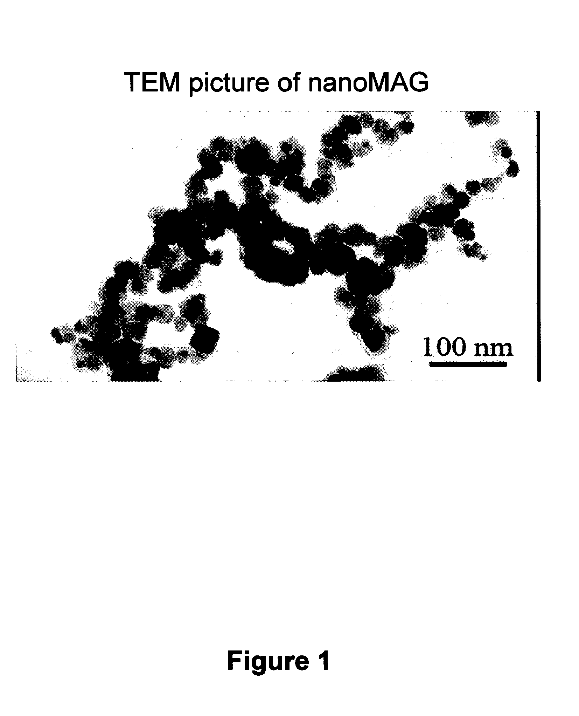

The magnetic core may further be coated with a shell, for example a silica shell. For this purpose, a first silica impregnation is realized by addition of sodium silicate in particles solution (780µL for 1g particles in 240mL distilled water). Remaining sodium silicate is removed by a centrifugation against water. 125 mg particles are dispersed in water / ethanol (1/4) solution containing 0.6 mmoles of tetraethylorthosilicate. Silica precursor hydrolyzation and condensation are enhanced by injection of ammonium solution in the bulk. Solution is incubated overnight before particles washing by centrifugation in distilled water. Coated particles are kept in water (pH is adjusted at about 7.4). The figure 1 stands for a transmission electronic microscopy picture of non targeted nanoMAG.

### EXAMPLE 2

This embodiment verifies the Maximum Tolerated Dose (MTD) of untargeted magnetic nanoparticles (untargeted NanoMAG) in healthy male and female C57BU6 mice and in healthy male and female Swiss Nude mice.

A Maximum Tolerated Dose (MTD) experiment was conducted on 12 healthy male and 12 healthy female C57BU6 mice as well as 3 healthy male and 3 healthy female Swiss Nude mice. They received a single IV bolus injection of the NanoMAG vehicle and the untargeted NanoMAG composition at different doses chosen by the Inventors.

The tolerance study was conducted as described in table 1 below:

| Group | Strain | Sex | Nb of Mice | Treatment | Dose (mg)/mouse | Nb of treatments |
|---|---|---|---|---|---|---|
| 1 | C57BL/6 | Male | 3 | Vehicle | | 1 |
| 2 | C57BL/6 | Male | 3 | untargeted NanoMAG | 0,006 | 1 |
| 3 | C57BL/6 | Male | 3 | untargeted NanoMAG | 0,03 | 1 |
| 4 | C57BL/6 | Male | 3 | untargeted | 0,06 | 1 |
| | | | | NanoMAG | | |
| 5 | C57BL/6 | Female | 3 | Vehicle | | 1 |
| 6 | C57BL/6 | Female | 3 | untargeted NanoMAG | 0,006 | 1 |
| 7 | C57BL/6 | Female | 3 | untargeted NanoMAG | 0,03 | 1 |
| 8 | C57BL/6 | Female | 3 | untargeted NanoMAG | 0,06 | 1 |
| 9 | Swiss Nude | Male | 3 | untargeted NanoMAG | 0,06 | 1 |
| 10 | Swiss Nude | Female | 3 | untargeted NanoMAG | 0,06 | 1 |

Body weight, clinical signs of toxicity and survival were recorded twice a week. A 25% loss of the body weight and/or animal death were considered as the criteria for toxicity.

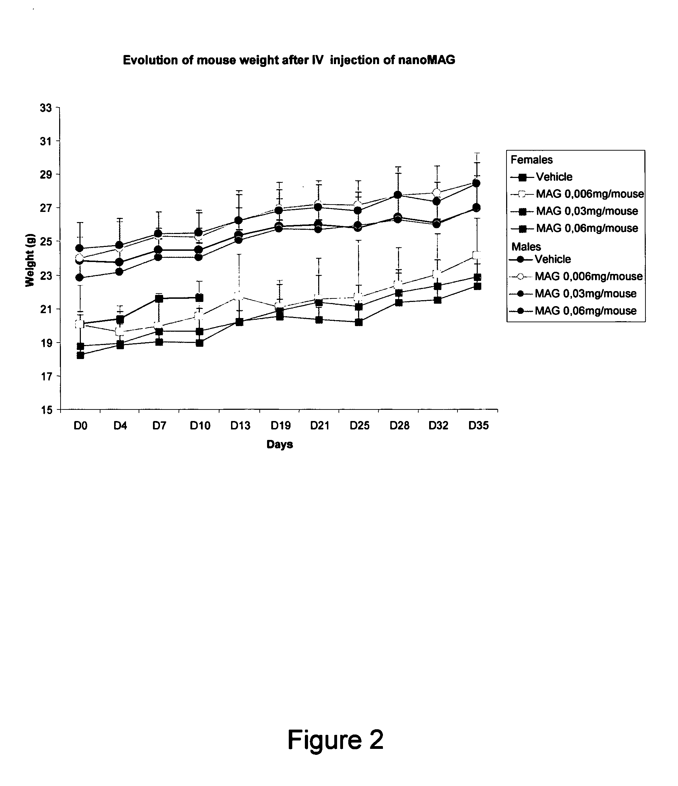

Figure 2 does not show any effect of the untargeted nanoMAG composition on mice weight, during the month following the administration of increasing doses thereof (ranging from 0,006 to 0,06 mg/mouse).
No dependency on mouse strain or mouse sex has been found.
Sacrifice and macroscopic autopsies of mice revealed no sign of toxicity.

### EXAMPLE 3

This embodiment further demonstrates the tolerance of healthy mice to untargeted magnetic nanoparticles (untargeted NanoMAG) after a single IV injection.

### 3.1 Material

- untargeted NanoMAG compositions: 3 g untargeted NanoMAG composition/L and 6 g untargeted NanoMAG composition/L
- 6 healthy C57BL/6 male mice

### 3.2 Treatment

- Administration route: IV, bolus
- Injection volume: 300µl/mouse/inj.
- Treatment doses: 0,90 and 1,80 mg untargeted NanoMAG composition/mouse/inj.
- Treatment schedule: Q1Dx1

Tolerance experiments including single injection of 0,9 and 1,8 mg / untargeted NanoMAG composition / mouse were achieved as described in table 2 below:

| No group | Strain | Sex | No mice | Test Substance | Treatment dose (mg of untargeted NanoMAG composition/ mouse/inj.) | Treatment schedule | Adm. route | Volume of adm /mouse (µl/mouse) |
|---|---|---|---|---|---|---|---|---|
| 1 | C57B L/6 | male | 3 | untargeted NanoMAG composition 3g/L | 0,90 | Q1Dx1 | IV | 300 |
| 2 | C57B L/6 | male | 3 | untargeted NanoMAG composition 6g/L | 1,80 | Q1Dx1 | IV | 300 |

Group 2 was injected only if Group 1 tolerated the 0,90 mg of untargeted NanoMAG composition / mouse/ inj. Dose.

### 3.3 Monitoring of mice

- body weight recorded twice a week for 10 days
- viability and behaviour recorded every day
- sacrifice and autopsy of mice achieved 10 days after the last injection

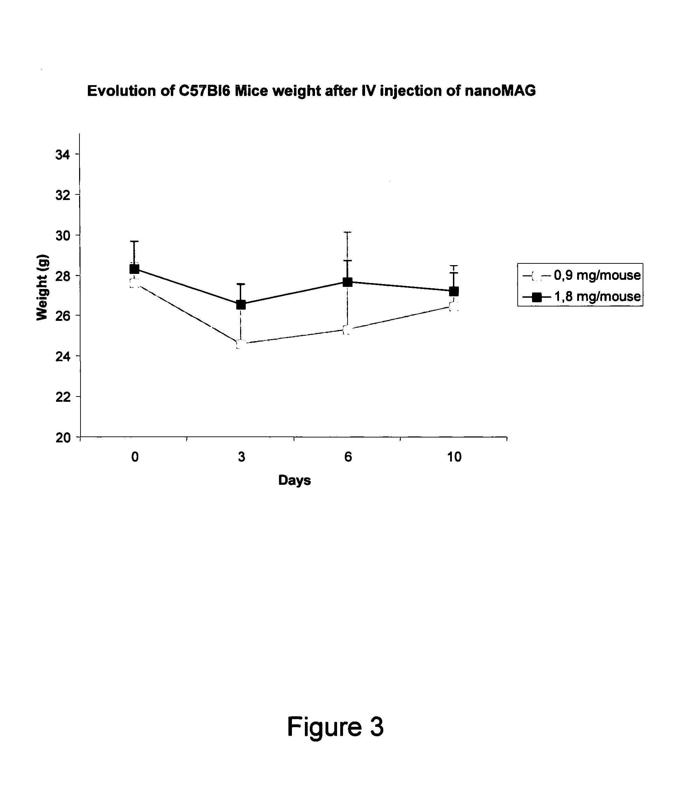

Tolerance experiments demonstrated no effect of untargeted nanoMAG composition on the mice weight (see Fig. 3).

### EXAMPLE 4

This embodiment demonstrates the tolerance of healthy C57BL6 and Swiss mice to untargeted magnetic nanoparticles (untargeted NanoMAG) after repeated injections.

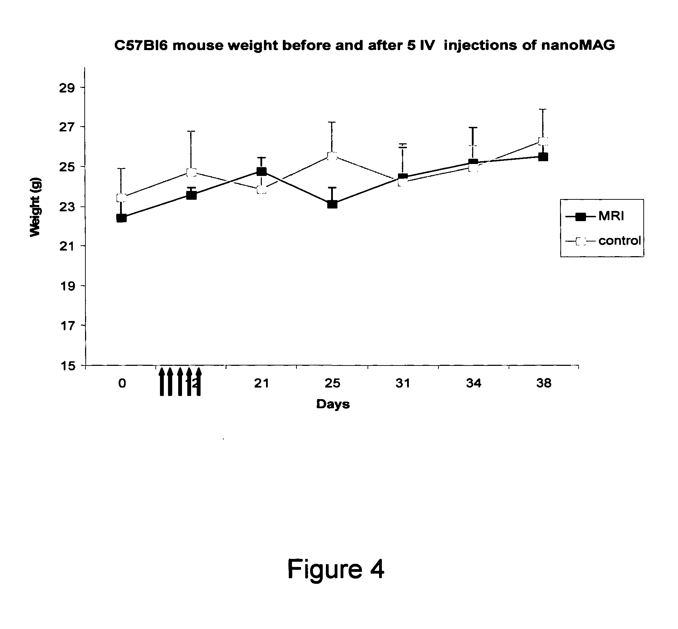

Similar experiments as those described in examples 2-3 have been achieved after repeated injections of untargeted nanoMAG. The mice weight, before and after 5 intravenous injections of untargeted nanoMAG composition (0.12mg/mouse), was measured (see Fig. 4). In order to verify the safety of nanoparticles after activation, some mice were subjected to a magnetic filed (MRI) to activate the nanoparticles. There was no difference between the weight of control mice and the weight of mice subjected to MRI. Moreover sacrifice and autopsies of animals revealed no sign of toxicity.

### EXAMPLE 5

This embodiment demonstrates the efficacy of untargeted NanoMAG compositions in female C57BL6 mice bearing C38 colon tumors.

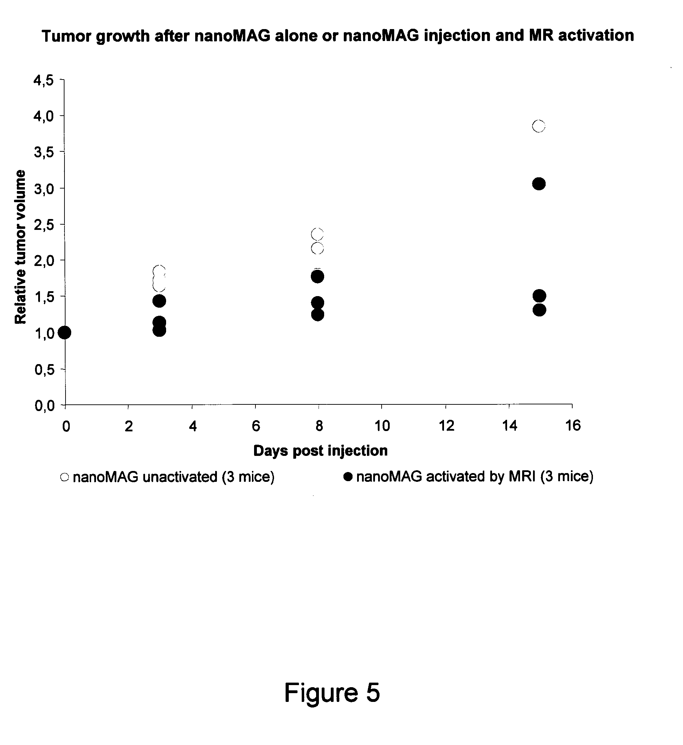

An untargeted NanoMAG composition (1.5g/l) was directly injected in C38 tumors beard by C57BI6 mice. The animals were subjected to a magnetic field (MRI) during 1 hour, 20 hours post-injection and then compared to untargeted nanoMAG injected and not activated animals. Figure 5 shows a decrease of the tumor growth in activated animals.

### EXAMPLE 6

This embodiment evaluates the antitumor activity of untargeted untargeted NanoMAG activated by a magnetic field, in a model of C38 tumor bearing C57BL6 male mice, after a single intratumoral (IT) injection, and under different conditions of delay between IT injection and magnetic field exposure.

The day of treatment (D0), 26 out of 50 tumor bearing C57BU6 male mice were randomized into 5 groups (1 group of 6 mice and 4 groups of 5 mice). The groups mean tumor volumes were not different (analysis of variance). The treatment schedule was chosen by inventors as follows:
- Group 1: five mice received a single IT injection of NaCl 0,9% solution (treatment schedule Q1Dx1).
- Group 2: Six mice received a single IT injection of untargeted NanoMAG at 0.75 mg of untargeted NanoMAG / ml of tumor (treatment schedule Q1Dx1).
- Group 3: five mice received a single IT injection of untargeted NanoMAG at 0.75 mg of untargeted NanoMAG / ml of tumor (treatment schedule Q1Dx1). The mice were then subjected during one hour to a 4,7 tesla magnetic field, 5 hours after IT injection of untargeted NanoMAG.
- Group 4: five mice received a single IT injection of untargeted NanoMAG at 0.75 mg of untargeted NanoMAG / ml of tumor (treatment schedule Q1Dx1). The mice were then subjected during one hour to a 4,7 tesla magnetic field, 20 hours after IT injection of untargeted NanoMAG.
- Group 5: five mice received a single IT injection of untargeted NanoMAG at 0.75 mg of untargeted NanoMAG / ml of tumor (treatment schedule Q1Dx1). The mice were then subjected during one hour to a 4,7 tesla magnetic field, 48 hours after IT injection of untargeted NanoMAG.

The treatment schedule is summarized in table 3 below:

| No group | Strain | Sex | No mice | Test Substance | Treatment schedule | Adm. route | Injected dose (mg untargeted NanoMAG/ml of tumor) |
|---|---|---|---|---|---|---|---|
| 1 | C57BL/6 | male | 5 | NaCl 0,9% | Q1Dx1 | IT | - |
| 2 | C57BL/6 | male | 6 | untargeted NanoMAG | Q1Dx1 | IT | 0,75 |
| 3 | C57BL/6 | male | 5 | untargeted NanoMAG | Q1Dx1 | IT | 0,75 |
| 4 | C57BL/6 | male | 5 | untargeted NanoMAG | Q1Dx1 | IT | 0,75 |
| 5 | C57BL/6 | male | 5 | untargeted NanoMAG | Q1Dx1 | IT | 0,75 |

The exposure of mice to the magnetic field and activation was performed as described in table 4 below:

| Groups | No Mice | Test Substance | Activation duration | Time of activation after injection | Imaging |
|---|---|---|---|---|---|
| 1 | 5 | NaCl 0,9% | None | None | Yes |
| 2 | 6 | untargeted NanoMAG | None | None | None |
| 3 | 5 | untargeted NanoMAG | 1H | 5H | Yes |
| 4 | 5 | untargeted NanoMAG | 1 H | 20H | Yes |
| 5 | 5 | untargeted NanoMAG | 1H | 48H | Yes |

Body weight, clinical signs of toxicity and survival were recorded twice a week. A 15-20% loss of the body weight and/or animal death were considered as the criteria for toxicity.

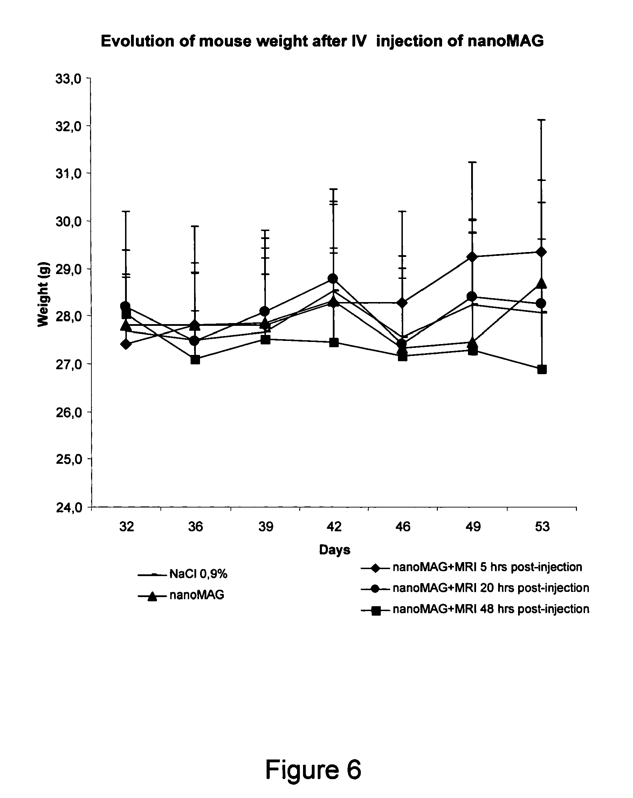

This experience revealed no difference of weight between control and treated animals (see Fig.6).

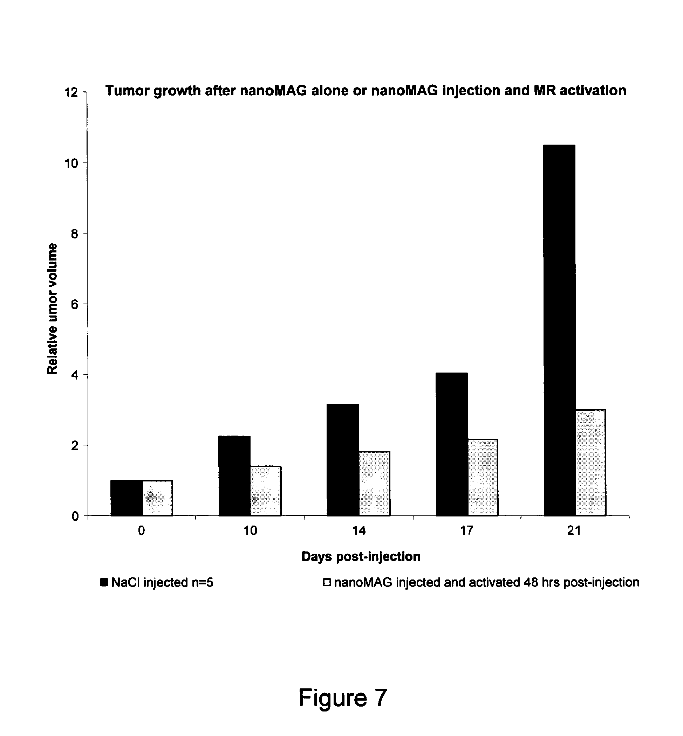

The length and width of the tumor was measured with calipers and the volume of the tumor was estimated by the formula (width² × length)/2. Treatment efficacy was assessed in terms of the effects of activated untargeted NanoMAG on the tumor volumes of treated mice relative to untreated mice. The tumor growth was monitored and is represented on Fig. 7. The growth of the tumors is decreased when mice are treated with untargeted NanoMAG activated 48h after their injection.

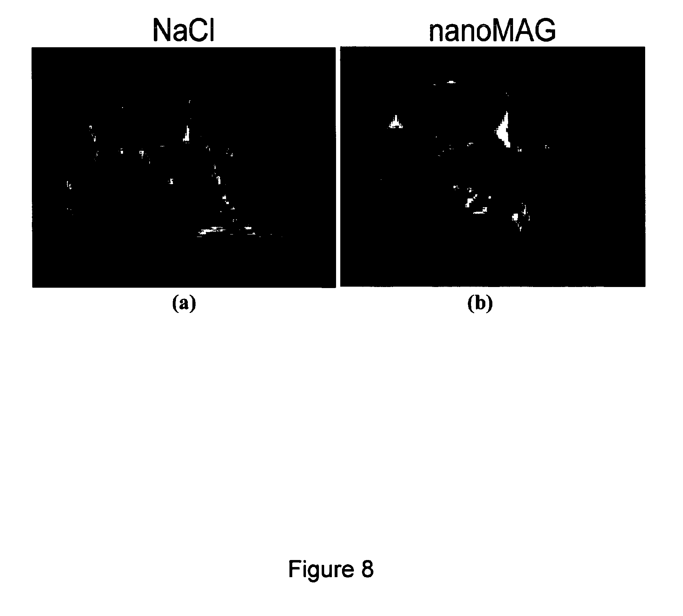

FIG. 8 shows an MRI pictures of mice, bearing C38 tumors injected intra tumorousy with NaCl 0,9% (a) or with nanoMAG (b), 48 hours after injection. nanoMAG are detectable *in vivo* and lead to a specificity and a contrast enhancement.

## Claims

1. Use of a biocompatible nanoparticle or nanoparticle aggregate, in combination with an external non-oscillating magnetic field, wherein said nanoparticle comprises:
a) a core comprising ferromagnetic material; and
b) a biocompatible shell surrounding the core, wherein the outer diameter of the shell is less than about 100 nm;
for the manufacture of a pharmaceutical composition, to prevent or treat a cancer, wherein the composition is deprived of any cell targeting means.

2. Use according to claim 1, wherein the ferromagnetic material is selected from the group made of iron, nickel, cobalt, gadolinium, samarium, neodymium, boron, aluminium, and any mixture thereof.

3. Use according to claim 2, wherein the ferromagnetic core material is in the form of an oxide, an hydroxide or a metal.

4. Use according to claim 3, wherein the ferromagnetic material is a monodomain ferromagnetic material.

5. Use according to claim 4, wherein the magnetic core material is selected from the group consisting of ferrous oxide and ferric oxide.

6. Use according to anyone of claims 1 to 5, wherein the biocompatible shell is made of a material selected from the group consisting of silica, gold, alumina, **sugar, PEG and dextran.**

7. Use according to claim 6, wherein the outer diameter of the shell is between about 10 nm and less than about 100 nm.

8. Use according to claim 7, wherein the outer diameter of the shell is between about 10 nm and about 50 nm.

9. Use according to anyone of claim 1 to 8, wherein the nanoparticle further comprises a labelling agent attached to the magnetic material.

10. Use according to anyone of claim 1 to 9, wherein the nanoparticle or nanoparticle aggregate has an essentially spherical or round shape.

11. Use of a biocompatible nanoparticle or nanoparticle aggregate, in combination with an external magnetic field, wherein said nanoparticle comprises:
a) a core comprising ferromagnetic material;
b) a biocompatible shell surrounding the core, wherein the outer diameter of the shell is less than about 100 nm; and
c) a labelling agent;
for the manufacture of a monitoring composition intended for the treatment and detection or the visualization of a cancer cell, tissue or organ, wherein the composition is deprived of any cell targeting means.

12. Use according to anyone of claims 1 to 11, wherein the magnetic field is applied by using any magnetic field source for generating therapeutic activity.

13. Use according to claim 12, wherein the magnetic field source is a uniform and unidirectional magnetic field source.

14. Use according to claim 12 or 13, wherein the magnetic field source is selected from any electromagnet and Magnetic Resonance Imaging (MRI) equipment.

15. Use according to claim 11, wherein the scanner or radiography or ultrasound is use for visualization purpose.

16. A pharmaceutical composition, deprived of any cell targeting agent, comprising a biocompatible nanoparticle or nanoparticle aggregate comprising a) a core comprising ferromagnetic material, and b) a biocompatible shell surrounding the core, wherein the outer diameter of the shell is less than about 100 nm, in association with a biocompatible carrier.

17. Composition according to claim 16, wherein the ferromagnetic core material is selected from the group consisting of ferrous oxide and ferric oxide.

18. Composition according to claim 16 or 17, wherein the outer diameter of the shell is between about 10 nm and about 50 nm.

19. Composition according to anyone of claims 16 to 18, wherein the nanoparticle or nanoparticle aggregate has an essentially spherical or round shape.

20. A diagnostic composition, deprived of any cell targeting agent, comprising a biocompatible nanoparticle or nanoparticle aggregate comprising a) a core comprising ferromagnetic material; b) a biocompatible shell surrounding the core, and c) a labelling agent, wherein the outer diameter of the shell is less than about 100 nm and preferably less than 50nm, in association with a biocompatible carrier.

21. Composition according to claim 20, wherein the ferromagnetic core material is selected from the group consisting of ferrous oxide and ferric oxide.

22. Composition according to claim 20 or 21, wherein the outer diameter of the shell is between about 10 nm and about 50 nm.

23. Composition according to anyone of claims 20 to 22, wherein the nanoparticle or nanoparticle aggregate has an essentially spherical or round shape.
